# EUROPEAN PATENT SPECIFICATION

(11) **EP 3 320 945 B1**
(45) Date of publication and mention of the grant of the patent: **18.09.2019**
(21) Application number: 16821427.8
(22) Date of filing: 06.07.2016
(51) Int. Cl.: A61M 37/00, B81B 1/00, A61K 9/70, A61K 9/00

(54) **PERCUTANEOUS ADMINISTRATION DEVICE**
VORRICHTUNG FÜR PERKUTANE VERABREICHUNG
DISPOSITIF D'ADMINISTRATION PERCUTANÉE

(30) Priority: 07.07.2015 JP 2015136286
(43) Date of publication of application: 16.05.2018
(73) Proprietor: Toppan Printing Co., Ltd., Tokyo 110-0016 (JP)
(72) Inventor: UENO, Hisami, Tokyo 110-0016 (JP)
(74) Representative: TBK
(86) International application number: PCT/JP2016/069994
(87) International publication number: WO 2017/006958

(56) References cited:
- EP-A1- 1 385 427
- WO-A1-2010/070628
- WO-A1-2015/009531
- WO-A1-2015/012252
- JP-A- 2003 531 404
- JP-A- 2010 075 374
- JP-A- 2013 193 268
- US-A1- 2012 136 312
- US-A1- 2013 285 361
- US-B2- 7 537 590

## Description

### [Technical Field]

The present invention relates to transdermal administration devices used for drug administration.

### [Background Art]

A transdermal administration device includes an administration section for administering a drug through the skin of a drug administration target. A microneedle, which is an example of the administration section, includes a plurality of needle-shaped projections and a plate-shaped substrate. The plurality of projections are arranged on the surface of the substrate. In a drug administration method using the microneedle, the substrate is first pressed against the skin of the administration target to pierce the skin using the projections. Then, a drug is introduced into the body of the administration target through the holes created by the projections. Such a microneedle is formed of, for example, a water-soluble resin, a biocompatible resin such as a biodegradable resin, or the like (for example, see WO 2006/080508 A1 and JP 2005-21677 A).

US 2012/136312 A1, EP 1 385 427 A1 or WO 2010/070628 A1 relate to similar transdermal administration devices.

### [Summary of the Invention]

### [Technical Problem]

The microneedle formed of the aforementioned material is, for example, a colorless transparent structure and is usually light transparent. The microneedle having such a configuration is not readily visible, and for example, it is difficult to recognize the position of the microneedle on the support member where the microneedle is placed.

One of the techniques of enhancing the visibility of the microneedle is to provide a coloring agent in the microneedle. However, materials forming the microneedle do not always have chemical properties that can be mixed with a coloring agent. When the administration target is a living body, the coloring agent must be biocompatible. Further, when the administration target is a human, coloring of the skin by the coloring agent is not desired. The coloring agents that meet these requirements are limited.

Therefore, there is a need of enhancing the visibility of the administration section such as a microneedle in a transdermal administration device taking advantage of a structural feature.

The present invention aims to provide a transdermal administration device that is readily visible.

### [Solution to Problem]

A transdermal administration device, as described in the claims, for solving the above problem includes: a substrate having a first surface and a second surface which is a surface opposite to the first surface; a plurality of projections protruding from the first surface, the plurality of projections being provided for piercing an administration target; and a raised and/or recessed structure composed of a plurality of raised and/or recessed elements, the plurality of raised and/or recessed elements including at least either of raised elements raised from the second surface or recessed elements recessed from the second surface, wherein the substrate, the projections and the raised and/or recessed structure are made of transparent material, and the plurality of raised and/or recessed elements forms a pattern as viewed in a direction perpendicular to the second surface which is finer than a pattern formed by the plurality of projections as viewed in a direction perpendicular to the first surface.

According to the above configuration, since the raised and/or recessed structure composed of the plurality of fine raised and/or recessed elements are disposed, light incident on the transdermal administration device undergoes diffuse reflection at the position of the raised and/or recessed elements to thereby change the propagation direction of the light to various directions. As a result, the raised and/or recessed structure looks different from the other portion, which improves visibility of the transdermal administration device. Further, when the transdermal administration device is configured to be transparent, visibility is particularly low if the transdermal administration device does not include the raised and/or recessed structure. Therefore, it is particularly advantageous to provide the raised and/or recessed structure to improve visibility.

In the above transdermal administration device, it is preferred that a distance between the respective ends of two of the projections adjacent to each other as viewed in the direction perpendicular to the first surface, which is the distance between the respective ends oriented in the same direction in a direction of an array of the projections, is an inter-projection distance, and a distance between the respective ends of two of the raised and/or recessed elements adjacent to each other as viewed in the direction perpendicular to the second surface, which is the distance between the respective ends oriented in the same direction in a direction of an array of the raised and/or recessed elements, is an inter-raised and/or recessed element distance, and the inter-raised and/or recessed element distance is smaller than the inter-projection distance.

According to the above configuration, a pattern formed by the plurality of raised and/or recessed elements can be appropriately formed to be finer than a pattern formed by the plurality of projections.

In the above transdermal administration device, it is preferred that a maximum length of each of the raised and/or recessed elements in a direction parallel with the second surface is smaller than a maximum length of each of the projections in a direction parallel with the first surface.

According to the above configuration, a pattern formed by the plurality of raised and/or recessed elements can be appropriately formed to be finer than a pattern formed by the plurality of projections.

In the above transdermal administration device, it is preferred that an area occupied by one of the raised and/or recessed elements as viewed in the direction perpendicular to the second surface is smaller than an area occupied by one of the projections as viewed in the direction perpendicular to the first surface.

According to the above configuration, a pattern formed by the plurality of raised and/or recessed elements can be appropriately formed to be finer than a pattern formed by the plurality of projections.

In the above transdermal administration device, it is possible that a maximum length of each of the raised and/or recessed elements in a thickness direction of the substrate is smaller than a maximum length of each of the projections in a thickness direction of the substrate.

According to the above configuration, the size of the raised and/or recessed elements in the thickness direction of the substrate is prevented from being excessively increased. As a result, an increase in load for manufacturing the raised and/or recessed elements can be reduced.

In the above transdermal administration device, the plurality of raised and/or recessed elements may be a diffraction grating.

In the above configuration, visibility of the transdermal administration device is appropriately improved since the raised and/or recessed structure 13 exhibits a structural color.

The transdermal administration device may further include an adhesive sheet having an adhesive layer bonded to the second surface of the substrate, wherein the adhesive layer extends outward from the substrate as viewed in a direction perpendicular to the first surface of the substrate.

According to the above configuration, visibility of a portion which serves as an administration section in the transdermal administration device having the adhesive sheet is improved.

### [Advantageous Effects of Invention]

According to the present invention, visibility of the transdermal administration device can be improved.

### [Brief Description of the Drawings]

Fig. 1 is a perspective view that illustrates a perspective structure of a microneedle which is an example of an administration section in a transdermal administration device according to one embodiment of a transdermal administration device.
Fig. 2 is a perspective view which illustrates a perspective structure of a microneedle according to one embodiment.
Fig. 3 is a cross-sectional view which illustrates a cross-sectional structure of an example of a microneedle according to an embodiment.
Fig. 4 is a cross-sectional view which illustrates a cross-sectional structure of an example of a microneedle according to one embodiment.
Fig. 5 is a plan view which illustrates a planar structure of a microneedle according to one embodiment.
Fig. 6 is a plan view which illustrates an arrangement position of a raised and/or recessed area in an example of a microneedle according to one embodiment.
Fig. 7 is a plan view which illustrates an arrangement position of a raised and/or recessed area in an example of a microneedle according to one embodiment.
Fig. 8 is a plan view which illustrates an arrangement position of a raised and/or recessed area in an example of a microneedle according to one embodiment.
Fig. 9 is a plan view which illustrates an arrangement position of a raised and/or recessed area in an example of a microneedle according to one embodiment.
Fig. 10 is a plan view which illustrates an arrangement position of a raised and/or recessed area in an example of a microneedle according to one embodiment.
Fig. 11 is a cross-sectional view which illustrates a cross-sectional structure of a microneedle according to a first modified example.
Fig. 12 is a cross-sectional view which illustrates a cross-sectional structure of a transdermal administration device of a second modified example.
Fig. 13 is a cross-sectional view which illustrates a cross-sectional structure of a microneedle according to a modified example.

### [Description of Embodiments]

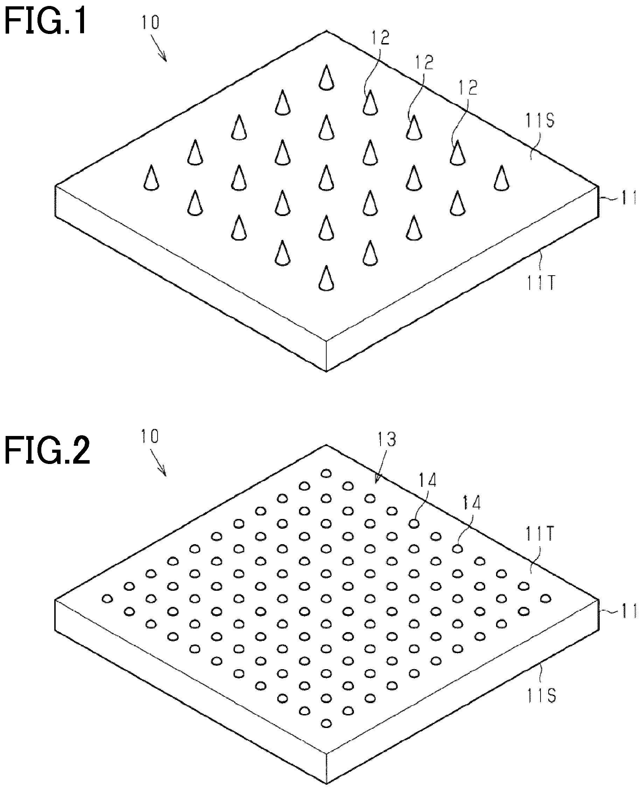

With reference to Figs. 1 to 10, an embodiment of a transdermal administration device will be described.

### [Configuration of Transdermal Administration Device]

With reference to Figs. 1 to 10, a configuration of a microneedle, which is an example of an administration section that constitutes a transdermal administration device, will be described.

As shown in Fig. 1, a microneedle 10 includes a plate-shaped substrate 11 and a plurality of projections 12 protruding from the substrate 11. The substrate 11 has a first surface 11S on which the projections 12 are formed and a second surface 11T which is a surface opposite to the first surface 11S. The first surface 11S supports the proximal ends of the projections 12.

The outer shape of the substrate 11 as viewed in the direction perpendicular to the first surface 11S is not specifically limited, and may be a circle, oval or rectangle.

Each of the projections 12 may be a cone shape or a pyramid shape. Further, each of the projections 12 may be a shape which does not have a pointed tip, for example, a cylinder or prism shape. Further, each of the projections 12 may be a shape composed of a combination of two or more three dimensional shapes, for example, a cone stacked on a cylinder. In other words, each of the projections 12 may be any shape that can pierce the skin. Moreover, each of the projections 12 may have a narrow portion or shoulder formed on the side wall, or a hole extending in an extending direction of the projections 12. Further, the plurality of projections 12 may include the projections 12 having different shapes from each other.

The plurality of projections 12 may be arranged regularly or irregularly as viewed in the direction perpendicular to the first surface 11S of the substrate 11. For example, the plurality of projections 12 is arranged in a matrix or concentric pattern. When viewed in the direction perpendicular to the first surface 11S of the substrate 11, the plurality of projections 12 forms a pattern of regularly or irregularly arranged projections 12.

As shown in Fig. 2, the microneedle 10 further includes a raised and/or recessed structure 13 on the surface of the substrate 11 opposite to the projections 12. The raised and/or recessed structure 13 is composed of a plurality of raised and/or recessed elements 14, and the plurality of raised and/or recessed elements 14 include at least either of raised elements raised from the second surface 11T or recessed elements recessed from the second surface 11T. That is, the raised and/or recessed structure 13 is composed of a plurality of raised elements, a plurality of recessed elements, or the raised elements and the recessed elements. Fig. 2 illustrates an example where the plurality of raised and/or recessed elements 14 is composed of the raised elements.

The shape of each of the raised and/or recessed elements 14, that is, the shape of the raised element and the shape of the recessed element are not specifically limited. For example, the raised element may be a hemisphere shape, a pyramid shape such as a cone and a polygonal pyramid, a columnar shape such as a circular column and a polygonal column, or any three dimensional shape other than those included in the above shapes. Further, the three dimensional shape surrounded by the inner wall of the recessed element, that is, the shape of the recess may be a hemisphere shape, a pyramid shape, a columnar shape, or any three dimensional shape other than those included in the above shapes. Further, the plurality of raised and/or recessed elements 14 may include a plurality of raised elements having shapes different from each other or a plurality of recessed elements having shapes different from each other.

The plurality of raised and/or recessed elements 14 may be arranged regularly or irregularly as viewed in the direction perpendicular to the second surface 11T of the substrate 11. When viewed in the direction perpendicular to the second surface 11T of the substrate 11, the plurality of raised and/or recessed elements 14 forms a pattern of regularly or irregularly arranged raised and/or recessed elements 14.

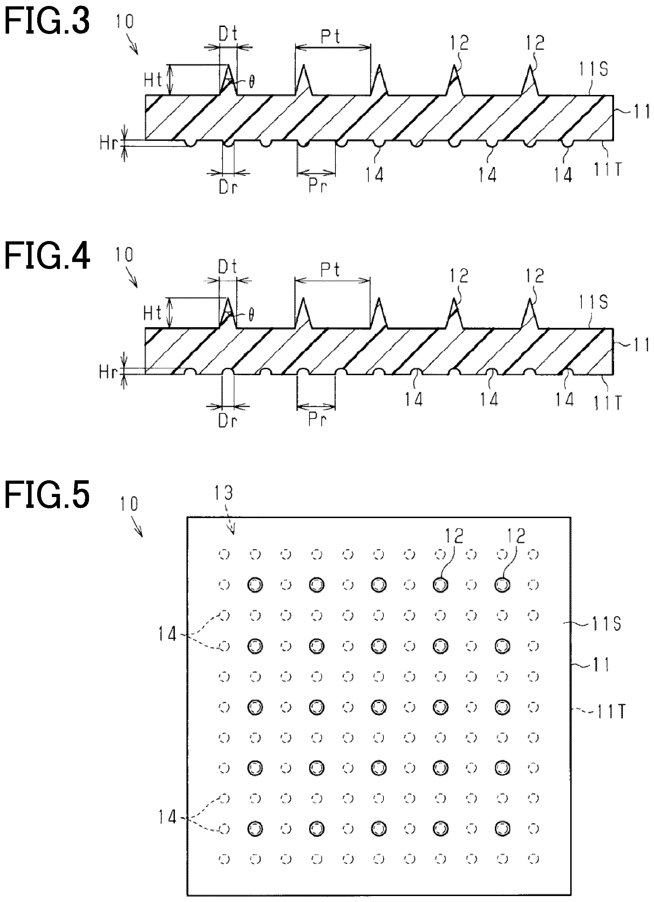

With reference to Figs. 3 to 5, the respective sizes of the projections 12 and the raised and/or recessed elements 14 will be described in detail. Fig. 3 illustrates an example where the plurality of raised and/or recessed elements 14 is solely composed of the raised elements, and Fig. 4 illustrates an example where the plurality of raised and/or recessed elements 14 is solely composed of the recessed elements. Fig. 5 is a view of the microneedle 10 as viewed in the direction perpendicular to the first surface 11S of the substrate 11, in which the outer shapes of the raised and/or recessed elements 14 disposed on the second surface 11T of the substrate 11 are indicated by the dotted line.

As shown in Fig. 3, each of the projections 12 has a length Ht, which is a maximum length of each of the projections 12 in the thickness direction of the substrate 11, that is, a direction perpendicular to the first surface 11S. The length Ht is a length from the first surface 11S of the substrate 11 to the tip of each of the projections 12. The length Ht of each of the projections 12 is preferably in the range of 20 µm or more and 2000 µm or less. The length Ht of each of the projections 12 is determined depending on the purpose of the puncture made by the projections 12, the type of drug administered, and a depth of hole of the puncture required to be created by the projections 12.

For example, when the puncture target is the human skin and the depth of the hole of the puncture created by the projection 12 is a length that passes through the stratum corneum and does not reach the nerve plexus, the length Ht of the projection 12 is preferably in the range of 200 µm or more and 700 µm or less, more preferably in the range of 200 µm or more and 600 µm or less.

When the depth of the hole is in such a range that penetrates the stratum corneum and does not reach nerve plexus, the drug can be delivered to a site deeper than the stratum corneum. Since the hole formed in the stratum corneum closes as the time elapses, the stratum corneum serves as a barrier to the outside so that the drug delivered deeper than the stratum corneum is held in the body. Accordingly, the drug can be held in the body for a long period of time since the drug is prevented from being peeled off due to the metabolism of the stratum corneum or washing skin for skin care or the like.

Moreover, when the depth of the hole is in such a range that locates it within the stratum corneum, the length Ht of the projection 12 is preferably in the range of 30 µm or more and 300 µm or less, and more preferably in the range of 30 µm or more and 200 µm or less.

Since the depth of the hole is in a range that locates in the stratum corneum, the drug can be retained in the stratum corneum. The drug in the stratum corneum is excreted as the time elapses since the stratum corneum is constantly newly produced by metabolism. Accordingly, a state in which the drug retained in the body is easily released, for example, by washing the skin or peeling the skin.

The lengths Ht of the plurality of projections 12 may be the same or different from each other. For example, in the case where the length Ht of the projections 12 located on the outer periphery area among the plurality of projections 12 is larger than the length Ht of the projections 12 located in the center area, the projections 12 can be easily in contact with a curved surface of the skin when the skin of the administration target is a curved surface. Alternatively, for example, in the case where the length Ht of each of the projections 12 located on the outer periphery area among the plurality of projections 12 is smaller than the length Ht of each of the projections 12 located in the center area, the projections 12 located on the outer periphery area, which are susceptible to an external force, may have an improved mechanical strength.

Each of the projections 12 has a width Dt, which is a maximum length of each of the projections 12 in a direction parallel with the first surface 11S of the substrate 11. The width Dt of each of the projections 12 is determined depending on the required aspect ratio of each of the projections 12 or the required volume of the hole. The width Dt of each of the projections 12 is preferably in the range of 1 µm or more and 300 µm or less. For example, when the projection 12 has a cone shape or a circular columnar shape, the width Dt of the projection 12 is a diameter of a circle which is the bottom of the projection 12, that is, the bottom which is in contact with the first surface 11S of the substrate 11. Further, for example, when the projection 12 has a regular quadrangular pyramid shape or a regular quadrangular prism shape, the width Dt of the projection 12 is a length of a diagonal of the square which is the bottom of the projection 12 which is in contact with the first surface 11S of the substrate 11.

When the tip of the projection 12 is formed in a pointed shape and the hole of the puncture is formed to penetrate the stratum corneum, the tip angle θ of the projection 12 is preferably in the range of 5° or more and 45° or less, and more preferably in the range of 8° or more and 25° or less.

The tip angle θ of the projection 12 is a maximum angle made by the tip of the projection 12 in a cross section perpendicular to the first surface 11S of the substrate 11. For example, when the projection 12 has a regular quadrangular pyramid shape, the tip angle θ of the projection 12 is an apex angle of a triangle having a diagonal line of a square of the bottom of the projection 12 as a base and the apex of the regular quadrangular pyramid projection 12 as an apex.

Further, the shape of the projection 12 is not limited to the shape above described, and may be appropriately determined depending on the purpose of puncture made by the projection 12 or the type of drug administered. For example, the purpose of puncture made by the projection 12 may be promotion of percutaneous absorption of the drug or extraction of a substance in the body to the outside the body through the skin. Further, the shape of the projection 12 can be determined in view of improvement in piercing performance to the skin.

Each of the raised and/or recessed elements 14 has a length Hr, which is a maximum length of each of the raised and/or recessed elements 14 in the thickness direction of the substrate 11. The length Hr is a length from the second surface 11T to the end of each of the raised and/or recessed elements 14 in the thickness direction of the substrate 11. That is, as shown in Fig. 3, when the raised and/or recessed elements 14 are composed of the raised elements, the length Hr of each of the raised and/or recessed elements 14 is a length from the second surface 11T to the tip of each of the raised and/or recessed elements 14, that is, the highest point of the raised element in the thickness direction of the substrate 11. Further, as shown in Fig. 4, when the raised and/or recessed elements 14 are composed of the recessed elements, the length Hr of each of the raised and/or recessed elements 14 is a length from the second surface 11T to the bottom of each of the raised and/or recessed elements 14, that is, the deepest point of the recessed element in the thickness direction of the substrate 11.

The length Hr of each of the raised and/or recessed elements 14 is preferably smaller than the length Ht of each of the projections 12, and is set to be in the range of, for example, 0.05 µm or more and 1000 µm or less, and preferably in the range of 0.05 µm or more and 500 µm or less. When the plurality of projections 12 do not have the identical length Ht or when the plurality of raised and/or recessed elements 14 do not have the identical length Hr, the minimum value among the lengths Hr of the plurality of raised and/or recessed elements 14 should be smaller than the minimum value among the lengths Ht of the plurality of projections 12.

Each of the raised and/or recessed elements 14 has a width Dr, which is a maximum length of each of the raised and/or recessed elements 14 in a direction parallel with the second surface 11T of the substrate 11. The width Dr of each of the raised and/or recessed elements 14 is preferably smaller than the width Dt of each of the projections 12, and is set to be in the range of, for example, 0.05 µm or more and 1000 µm or less, and preferably in the range of 0.05 µm or more and 500 µm or less. When the plurality of projections 12 do not have the identical width Dt or when the plurality of raised and/or recessed elements 14 do not have the identical width Dr, the minimum value among the widths Dr of the plurality of raised and/or recessed elements 14 should be smaller than the minimum value among the widths Dt of the plurality of projections 12.

In the configuration in which the length Hr of each of the raised and/or recessed elements 14 is smaller than the length Ht of each of the projections 12 and the width Dr of each of the raised and/or recessed elements 14 is smaller than the width Dt of each of the projections 12, the volume of each of the raised and/or recessed elements 14, that is, the volume of the raised element or the volume of the recessed element is smaller than the volume of each of the projections 12.

The projections 12 have a pitch Pt, which is a distance between two adjacent projections 12, that is, between one of the projections 12 and another of the projections 12 located most adjacent to the one of the projections 12. The distance is taken between the respective ends of the projections 12 as viewed in the direction perpendicular to the first surface 11S, that is, the respective ends oriented in the same direction in the direction of the array of the projections 12. In other words, the pitch Pt of the projections 12 is the sum of the width of one of the projections 12 in the direction in which two adjacent projections 12 are arranged and a gap length between these two adjacent projections 12. When the plurality of projections 12 is arranged in a cyclic manner with an equal interval, the plurality of projections 12 forms a cyclic pattern when viewed in the direction perpendicular to the first surface 11S, and the pitch Pt is the length of each cycle.

The raised and/or recessed elements 14 have a pitch Pr, which is a distance between two adjacent raised and/or recessed elements 14, that is, between one of the raised and/or recessed elements 14 and another of the raised and/or recessed elements 14 located most adjacent to the one of the raised and/or recessed elements 14. The distance is taken between the respective ends of the raised and/or recessed elements 14 as viewed in the direction perpendicular to the second surface 11T, that is, the respective ends oriented in the same direction in the array direction of the raised and/or recessed elements 14. In other words, the pitch Pr of the raised and/or recessed elements 14 is the sum of the width of one of the raised and/or recessed elements 14 in the direction in which two adjacent raised and/or recessed elements 14 are arranged and a gap length between these two adjacent raised and/or recessed elements 14. When the plurality of raised and/or recessed elements 14 are arranged in a cyclic manner with an equal interval, the plurality of raised and/or recessed elements 14 form a cyclic pattern when viewed in the direction perpendicular to the second surface 11T, and the pitch Pr is the length of each cycle.

The distance between the respective raised and/or recessed elements, or the pitch Pr of the raised and/or recessed elements 14 is smaller than the distance between the respective projections, or the pitch Pt of the projections 12. When the plurality of projections 12 do not have the identical pitch Pt or when the plurality of raised and/or recessed elements 14 do not have the identical pitch Pr, the minimum value among the pitches Pr of the plurality of raised and/or recessed elements 14 should be smaller than the minimum value among the pitches Pt of the plurality of projections 12.

As shown in Fig. 5, the size of each of the raised and/or recessed elements 14 as viewed in the direction perpendicular to the second surface 11T of the substrate 11 is preferably smaller than the size of each of the projections 12 as viewed in the direction perpendicular to the first surface 11S of the substrate 11. That is, an area occupied by one of the raised and/or recessed elements 14 as viewed in the direction perpendicular to the second surface 11T is preferably smaller than an area occupied by one of the projections 12 as viewed in the direction perpendicular to the first surface 11S.

When the plurality of projections 12 do not have an identical size as viewed in the direction perpendicular to the first surface 11S or when the plurality of raised and/or recessed elements 14 do not have an identical size as viewed in the direction perpendicular to the second surface 11T, the minimum value among the sizes of the plurality of raised and/or recessed elements 14 should be smaller than the minimum value among the sizes of the plurality of projections 12.

The plurality of raised and/or recessed elements forms a pattern as viewed in a direction perpendicular to the second surface which is finer than a pattern formed by the plurality of projections as viewed in a direction perpendicular to the first surface. In other words, the number of the raised and/or recessed elements 14 included in a unit area as viewed in the direction perpendicular to the second surface 11T is larger than the number of the projections 12 included in a unit area as viewed in the direction perpendicular to the first surface 11S.

This configuration is achieved by the aforementioned configuration having the width Dr of each of the raised and/or recessed elements 14 smaller than the width Dt of each of the projections 12 or the aforementioned configuration having the distance between the raised and/or recessed elements smaller than the distance between the projections, or the configuration having the area of each of the raised and/or recessed elements 14 as viewed in the direction perpendicular to the second surface 11T smaller than the area of each of the projections 12 as viewed in the direction perpendicular to the first surface 11S.

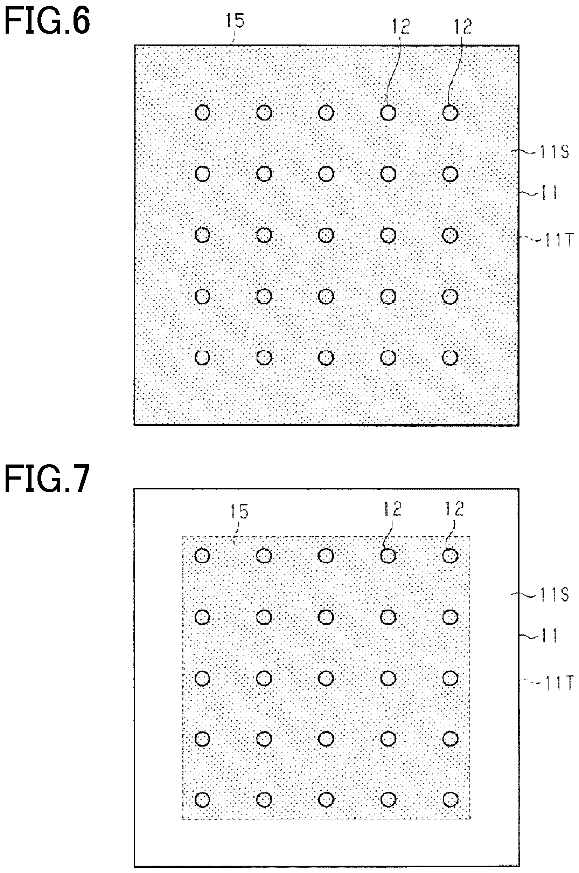

Referring now to Figs. 6 to 10, a position where the plurality of raised and/or recessed elements 14 is located on the second surface 11T, that is, a position where the raised and/or recessed structure 13 is located will be described. Figs. 6 to 10 are transparent diagrams of the substrate 11 as viewed in the direction perpendicular to the first surface 11S. A region of the second surface 11T where the raised and/or recessed structure 13 is located is indicated by dot hatching.

As shown in Fig. 6, the region where the raised and/or recessed structure 13 is located, which is a raised and/or recessed area 15, may extend over the entire surface of the second surface 11T.

Further, as shown in Fig. 7, the raised and/or recessed area 15 may be located only in a portion of the second surface 11T which overlaps with the region of the first surface 11S where a group composed of the plurality of projections 12 is located as viewed in the direction perpendicular to the first surface 11S.

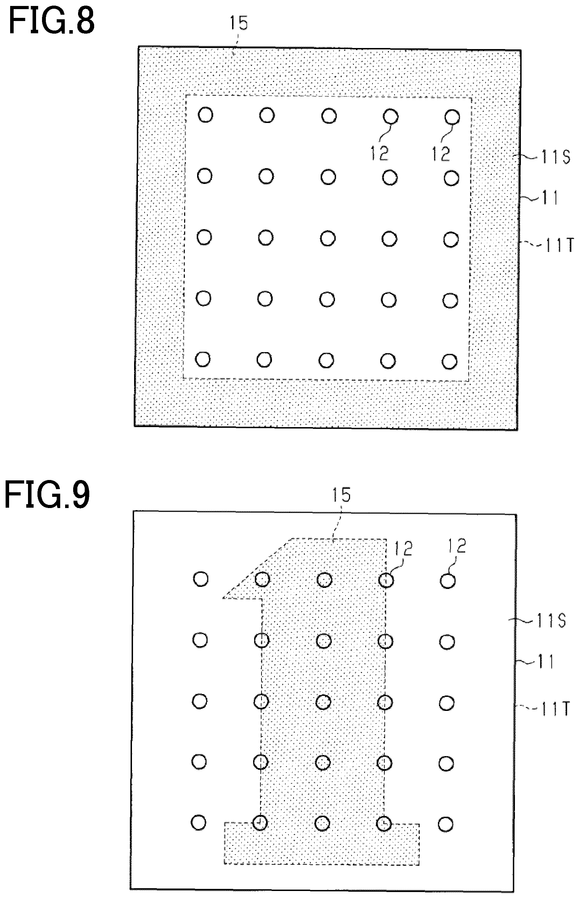

Further, as shown in Fig. 8, the raised and/or recessed area 15 may be located only in a portion of the second surface 11T which overlaps with outside the region of the first surface 11S where a group composed of the plurality of projections 12 is located as viewed in the direction perpendicular to the first surface 11S.

The raised and/or recessed structure 13 is more readily visible with increase in size of the raised and/or recessed area 15, and the effect of enhancing visibility of the microneedle 10 by means of the raised and/or recessed structure 13 is increased accordingly. Therefore, when the raised and/or recessed area 15 is located over the entire second surface 11T, the effect of enhancing visibility is increased.

Typically, the plurality of projections 12 is located at an easily recognizable position on the first surface 11S such as the center of the first surface 11S. In the configuration in which the raised and/or recessed area 15 overlaps with at least part of the region of the first surface 11S where a group composed of the plurality of projections 12 is located as viewed in the direction perpendicular to the first surface 11S, the raised and/or recessed structure 13 is readily identifiable since the raised and/or recessed structure 13 is located in a portion which overlaps with an easily recognizable position on the first surface 11S. Accordingly, visibility of the microneedle 10 is appropriately improved. Further, in the configuration in which the raised and/or recessed area 15 overlaps with the entire region of the first surface 11S where a group composed of the plurality of projections 12 is located as viewed in the direction perpendicular to the first surface 11S, the visibility of the microneedle 10 is further improved since the raised and/or recessed structure 13 is located at a more easily recognizable position.

Further, as shown in Fig. 9, the raised and/or recessed area 15 may be disposed on the second surface 11T, forming a design such as a number, character or symbol as viewed in the direction perpendicular to the first surface 11S.

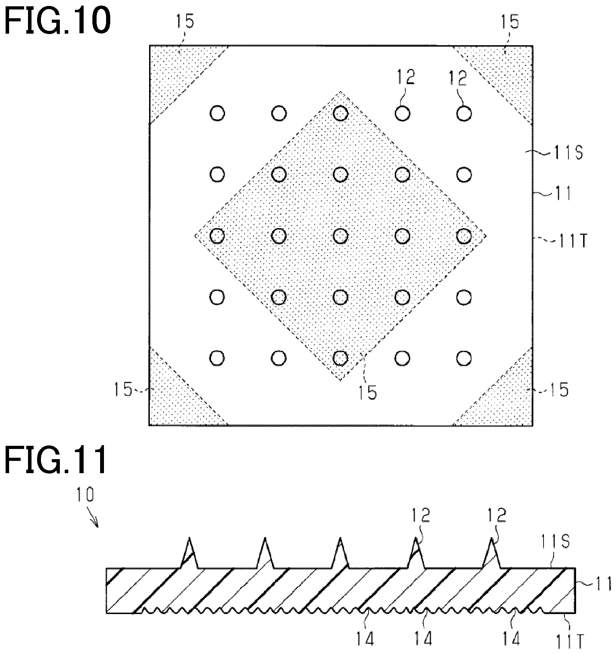

Further, as shown in Fig. 10, the raised and/or recessed area 15 may be disposed on the second surface 11T, forming a design such as a geometric shape, picture or pattern as viewed in the direction perpendicular to the first surface 11S. As shown in Fig. 10, the raised and/or recessed area 15 may include a plurality of regions distributed on the second surface 11T.

When the raised and/or recessed area 15 forms a design as viewed in the direction perpendicular to the first surface 11S, the raised and/or recessed structure 13 is readily identifiable. Accordingly, visibility of the microneedle 10 is appropriately improved.

The microneedle 10 composed of the substrate 11, the projections 12 and the raised and/or recessed structure 13 is made of a transparent material. In other words, the material of the microneedle 10 is transparent, and a portion of the microneedle 10 except for the raised and/or recessed structure 13 is transparent. Further, each of the raised and/or recessed elements 14 is transparent. The raised and/or recessed structure 13 which is composed of a group of the raised and/or recessed elements 14 has a lower transparency than the portion of the microneedle 10 other than the raised and/or recessed structure 13.

In the configuration having the transparent substrate 11 and projections 12, it is particularly advantageous to improve visibility by applying the configuration of the present embodiment since the visibility is particularly low if the configuration of the present embodiment is not applied. In the present embodiment, a transparent structure refers to a structure with a total light transmittance measured according to JIS K7361-1 (1997) of 50% or more and an internal haze measured according to JIS K7136 (2000) of 70% or less. Further, the internal haze of the microneedle 10 is measured by a technique such as covering the surface of the projections 12 and the raised and/or recessed structure 13 with a material having the same refractive index as that of the material forming the microneedle 10 to remove surface scattering of the microneedle 10.

The effect of enhancing the visibility is increased with increase in transparency of the microneedle 10. For this reason, it is preferred that a portion of the microneedle 10 except for the raised and/or recessed structure 13 has the total light transmittance of 70% or more and the internal haze of 50% or less.

The microneedle 10 is preferably made of a biocompatible material among the materials that can form the microneedle 10 and meet the requirements regarding the transparency. The biocompatible material has little effect on the body, and includes a water soluble polymer, water insoluble polymer, biopolymer, metal, resin and the like.

The biocompatible material may be a known material. Examples of the biocompatible material include alginates, curdlan, chitin, chitosan, glucomannan, polymalic acid, collagen, collagen peptide, hydroxypropyl cellulose, gelatin, silicon, titanium, silicone, polylactic acid, polyglycolic acid and the like. Although there is no clear distinction between chitin and chitosan, chitin with deacetylation of 70% or more is generally referred to as chitosan. Deacetylation may be performed by using a known technique. The chitin, chitosan, chitosan derivatives having biocompatibility may be substances originating from crustaceans such as crab and shrimp, substances originating from myceliums or microorganism generated plants, or substances using these as starting materials. The chitosan, chitin or chitosan, and chitin or chitosan derivatives are preferable as a forming material of the microneedle 10 since they have aesthetic effect on the skin as well as sterilization effect and antimicrobial effect.

The administration method using the microneedle 10 is not specifically limited. For example, a drug may be applied on the surface of the projections 12 so that the drug is delivered into the skin when the projections 12 pierce the skin. Alternatively, a drug may be contained in the projections 12 so that the drug is delivered into the skin when the projections 12 are dissolved while being pierced into the skin. Further, a liquid drug may be applied on the skin before or after the microneedle 10 is pierced into the skin so that the drug is delivered into the skin through the holes formed by the projections 12. Further, a liquid drug may be externally supplied to the microneedle 10 so that the drug is delivered into the skin through the projections 12. In this case, the microneedle 10 has a through hole that penetrates from the tip of the projection 12 to the second surface 11T of the substrate 11 so that the drug is delivered into the skin through the through hole. Moreover, a drug may be applied by combinations of these techniques. The projections 12 may be removed from the skin or may be embedded into the skin after being pierced into the skin.

The type of the drug is not specifically limited as long as it works when administered into the skin, and may be, for example, physiologically active agents or cosmetic compositions having aesthetic effect. Further, when an aromatic substance is used as a drug, a fragrance is imparted to the microneedle 10 to thereby obtain a microneedle 10 suitable for use as a beauty product. Moreover, the drug may include biologics. The biologics is a drug which uses a raw material or material derived from cells or cell tissues of a human or an animal.

As described above, the drug may be applied on the surface of the projections 12, contained in the projections 12, applied on the skin, or used in the form of a combination thereof depending on the method of drug administration.

The substrate 11 and the projections 12 may be made of a material having the same composition, or materials having different compositions. Further, when the raised and/or recessed elements 14 are composed of the raised elements, the substrate 11 and the raised and/or recessed elements 14 may be made of a material having the same composition, or materials having different compositions.

In the configuration in which the substrate 11 and the projections 12 are made of a material having the same composition, substrate 11 and the projections 12 are easily produced by integral molding, and in the configuration in which the substrate 11 and the raised and/or recessed elements 14 are made of a material having the same composition, substrate 11 and the raised and/or recessed elements 14 are easily produced by integral molding.

The microneedle 10 can be manufactured by using various known techniques. For example, when a resin is used as a material for the microneedle 10, the substrate 11 and the projections 12 can be produced by injection molding, extrusion molding, imprinting, hot embossing, casting or the like. Further, the substrate 11 and the projections 12 can be manufactured by machining such as cutting or by etching. Alternatively, an original plate of the microneedle may be formed to produce an intaglio plate having a reversed pattern of raised and recessed portions of the original plate by plating or by molding of a resin and thereby reproduce the microneedle 10 by using the produced intaglio plate.

The raised and/or recessed structure 13 may be produced by the same steps as those for producing the projections 12 or by different steps from those for producing the projections 12. Further, the projections 12 and the raised and/or recessed elements 14 may be formed by using the same processing technique or by using different processing techniques. Examples of processing techniques that can be used for producing the raised and/or recessed structure 13 include plasma treatment, sandblasting, nanoprinting, transfer molding, etching and laser processing.

### [Effects]

Effects of the transdermal administration device of the present embodiment will be described.

Since the microneedle 10 includes the raised and/or recessed structure 13 composed of the plurality of fine raised and/or recessed elements 14, optical properties of the microneedle 10 having the raised and/or recessed structure 13 are altered from those of the microneedle 10 which does not have the raised and/or recessed structure 13. Specifically, when light is incident on the raised and/or recessed elements 14, the light is scattered by diffuse reflection. Further, light diffracts, interferes or refracts at a position where the raised and/or recessed elements 14 are disposed depending of the shape of the raised and/or recessed elements 14.

With these configurations, the propagation direction of light transmitted through the microneedle 10 via the first surface 11S of the substrate 11 is changed into various directions at the position where the raised and/or recessed elements 14 is disposed. As a result, the raised and/or recessed structure 13 appears translucent when viewed in the direction facing the first surface 11S. Accordingly, the visibility of the microneedle 10 is improved. For example, the position of the microneedle 10 on the support member where the microneedle 10 is placed can be easily recognized, which improves handling of the microneedle 10. In particular, in the case of a small-sized microneedle 10 such as the case where the microneedle 10 is used as an alternative to injection needles so that a drug is administered via through holes formed in the projections 12, the present embodiment significantly contributes to improvement of the visibility of the microneedle 10 since the visibility is low if the raised and/or recessed structure 13 is not provided.

Further, since the raised and/or recessed structure 13 appears translucent when viewed in the direction facing the second surface 11T of the substrate 11, the visibility of the microneedle 10 is improved even if the second surface 11T is exposed.

As described above, when the microneedle 10 is made of a transparent material, the microneedle 10 can be further easily recognized due to improvement in visibility compared with the microneedle 10 which does not include the raised and/or recessed structure 13, thereby providing a feeling of safety to a user. In particular, the microneedle 10 of the present embodiment having the raised and/or recessed structure 13 which appears translucent due to light diffuse reflection or the like at the raised and/or recessed elements 14 provides an impression to a user that the microneedle is clean compared with the transparent microneedle 10 which does not include the raised and/or recessed structure 13. As a result, a user who is about to pierce the microneedle 10 to the skin has a feeling of safety before piercing the microneedle 10.

In the present embodiment, since the raised and/or recessed structure 13, which is a structure to improve visibility of the microneedle 10, is disposed on the surface of the substrate 11 opposite to the projections 12, visibility of the microneedle 10 can be improved without impairing a degree of freedom in positioning the projections 12, which is a portion that serves to administer a drug.

Further, since a pattern formed by the plurality of raised and/or recessed elements 14 is finer than a pattern formed by the plurality of projections 12, a portion of the microneedle 10 where the raised and/or recessed elements 14 are disposed has optical properties different from those of a portion where the projections 12 are disposed. Specifically, since a degree of change in light propagation direction attributed to the plurality of projections 12 is smaller than a degree of change in light propagation direction attributed to the plurality of raised and/or recessed elements 14, the portion where the plurality of projections 12 is disposed has a smaller degree of translucent than the portion where the raised and/or recessed structure 13 is disposed and has an outer appearance similar to a portion of the substrate 11 where the raised and/or recessed structure 13 is not disposed.

That is, providing the raised and/or recessed structure 13 causes a difference between the outer appearance of the microneedle 10 viewed in the direction perpendicular to the first surface 11S and the outer appearance of the microneedle 10 viewed in the direction perpendicular to the second surface 11T. Specifically, when viewed in the direction perpendicular to the first surface 11S, the portion which looks translucent due to the raised and/or recessed structure 13 is present on the rear side, while the portion where the projections 12 are disposed, which is a region that looks different from the translucent portion described above, is present on the front side. On the other hand, when viewed in the direction perpendicular to the second surface 11T, the portion which looks translucent due to the raised and/or recessed structure 13 is present on the front side.

As described above, since the microneedle 10 as viewed in the direction perpendicular to the first surface 11S is different from that as viewed in the direction perpendicular to the second surface 11T, the front and back sides of the microneedle 10 can be readily identified, that is, the surface of the substrate 11 on which the projections 12 are disposed can be readily identified. Accordingly, handling of the microneedle 10 is improved.

Further, since the microneedle 10 is readily visible and the front and back sides of the microneedle 10 can be readily identified as described above, the microneedle of the present embodiment is advantageous in visual inspection during manufacturing steps and the like compared with a transparent microneedle which does not include the raised and/or recessed structure 13. For example, according to the microneedle 10 of the present embodiment, the appearance of the projections 12 and the like can be easily inspected in visual inspection during manufacturing steps and whether or not a foreign substance is attached on the surface of the microneedle can be easily checked compared with a transparent microneedle which does not include the raised and/or recessed structure 13.

### [First Modified Example]

A first modified example of the transdermal administration device of the above embodiment will be described.

As shown in Fig. 11, the plurality of raised and/or recessed elements 14 may form a diffraction grating. For example, the raised and/or recessed elements 14 are strip-shaped raised elements or recessed elements, and the plurality of raised and/or recessed elements 14 are arranged with a predetermined space therebetween. In this configuration, the raised and/or recessed structure 13 exhibits a structural color due to interference of light at the raised and/or recessed structure 13, and the color of the portion where the raised and/or recessed structure 13 is present looks different depending on the direction it is viewed. Accordingly, visibility of the microneedle 10 is further improved.

Further, the diffraction grating can form a hologram which reproduces an image such as a character or a geometric shape. With this configuration, since a character or a geometric shape is visually observed at a position of the raised and/or recessed structure 13, visibility of the microneedle 10 is further improved. Further, by means of the way such a character or a geometric shape looks, the front and back sides of the microneedle 10 can be identified, that is, the surface of the substrate 11 on which the projections 12 are disposed can be identified. As a result, handling of the microneedle 10 is improved, and thus the safety in handling of the projections 12 is improved. In addition, individual identification of the microneedles 10 can be performed by taking advantage of the configuration of the raised and/or recessed structure 13, and such a configuration of the raised and/or recessed structure 13 can be used for enhancement of traceability or security.

Further, in the above configuration, the microneedle 10 may also include a layer for enhancing visibility of a color or image exhibited by the raised and/or recessed structure 13. For example, the microneedle 10 may include a reflective layer that covers the second surface 11T of the substrate 11 and reflects light incident on the substrate 11.

### [Second Modified Example]

A second modified example of the transdermal administration device of the above embodiment will be described. The transdermal administration device may include only the administration section as with the case of the above embodiment and the first modified example, or alternatively, may include other configurations in addition to the administration section.

The transdermal administration device of the second modified example includes an adhesive sheet in addition to the microneedle 10 which is an administration section.

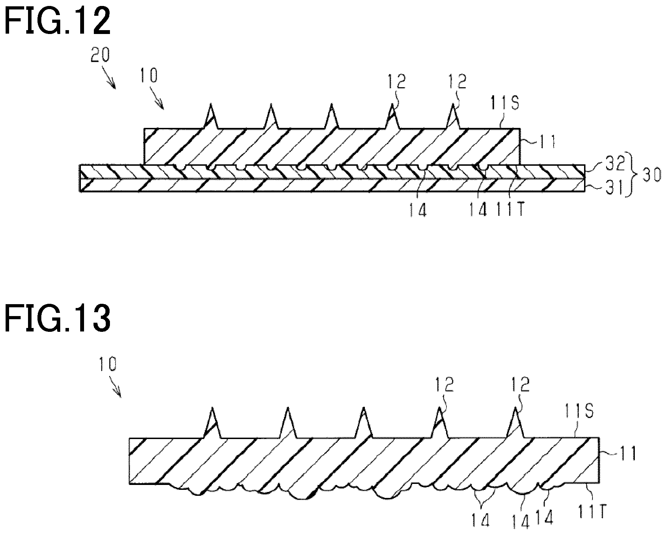

As shown in Fig. 12, a transdermal administration device 20 includes the microneedle 10 of the above embodiment and an adhesive sheet 30, and the adhesive sheet 30 includes a base sheet 31 and an adhesive layer 32 which covers one of two surfaces of the base sheet 31. The adhesive layer 32 is bonded to the second surface 11T of the substrate 11.

The outer shape of the adhesive sheet 30 is larger than the substrate 11 when viewed in the direction perpendicular to the first surface 11S of the substrate 11. In other words, the adhesive sheet 30 extends outward from the substrate 11 and the adhesive layer 32 is exposed when viewed in the direction perpendicular to the first surface 11S. The outer shape of the adhesive sheet 30 is not specifically limited, and the outer shape of the adhesive sheet 30 may be a circle, oval or rectangle.

The base sheet 31 and the adhesive layer 32 are preferably made of a material that does not disturb the effect of enhancing the visibility by means of the raised and/or recessed structure 13, and the material forming the adhesive layer 32 is preferably a biocompatible material. For example, in the configuration having the adhesive sheet 30 of a dark color, the raised and/or recessed structure 13 can be readily identified when viewed in the direction facing the first surface 11S.

The adhesive layer 32 preferably has an adhesive force that can hold the microneedle 10 and hold the adhesive sheet 30 to be adhered to the administration target for a desired period of time.

In use of the transdermal administration device 20, the substrate 11 is pressed against the skin of the administration target while a portion of the adhesive sheet 30 which extends outward from the substrate 11 is adhered to the skin of the administration target. As a result, the microneedle 10 is fixed to the skin of the administration target with the projections 12 being pierced into the skin.

With this configuration, visibility of the microneedle 10 in the transdermal administration device 20 having the adhesive sheet 30 is improved. Accordingly, for example, a position of the microneedle 10 on the adhesive sheet 30 can be easily recognized.

Moreover, the transdermal administration device may also include a protective sheet that covers the adhesive layer 32 of the adhesive sheet 30 which extends outward from the substrate 11 to protect the adhesive layer 32 and a lid that overlies the projections 12 of the microneedle 10 to protect the projections 12 in addition to the microneedle 10 and the adhesive sheet 30.

Further, the transdermal administration device may include an applicator that serves to assist piercing of the skin by the projections 12. For example, the applicator may include a portion that serves as a grip for a user when the user pierces the microneedle 10 into the skin, or a mechanism that imparts a biasing force to the projections 12 for puncturing the skin.

As described above, according to the transdermal administration device of the present embodiment and the modified examples, the effects listed below can be achieved.
(1) Since the raised and/or recessed structure 13 composed of the plurality of fine raised and/or recessed elements 14 is disposed on the surface of the substrate 11 opposite to the projections, light incident on the transdermal administration device is reflected by diffusion at the position of the raised and/or recessed elements 14 to thereby change the propagation direction of the light into various directions. As a result, the raised and/or recessed structure 13 looks different from other portions such as being translucent, which improves visibility of the transdermal administration device. Further, when the microneedle 10 is configured to be transparent, visibility is particularly low if the microneedle 10 does not include the raised and/or recessed structure 13. Therefore, it is particularly advantageous to provide the raised and/or recessed structure 13 to improve visibility.
(2) In the configurations in which the distance between the raised and/or recessed elements is smaller than the distance between the projections, the width Dr of the raised and/or recessed elements 14 is smaller than the width of the projections 12, or the area of the raised and/or recessed elements 14 as viewed in the direction perpendicular to the second surface 11T is smaller than the area of the projections 12 as viewed in the direction perpendicular to the first surface 11S, a pattern formed by the plurality of raised and/or recessed elements 14 can be appropriately formed to be finer than a pattern formed by the plurality of projections 12.
(3) In the configuration in which the length Hr of each of the raised and/or recessed elements 14 is smaller than the length Ht of each of the projections 12, the size of the raised and/or recessed elements 14 in the thickness direction of the substrate 11 is prevented from being excessively increased. That is, an increase in load for manufacturing the raised and/or recessed elements 14 can be reduced by preventing the size of the raised and/or recessed elements 14 in the thickness direction of the substrate 11 from being excessively increased since the raised and/or recessed elements 14 do not have a puncturing function for drug administration, unlike the projections 12.
(4) In the configuration in which the plurality of raised and/or recessed elements 14 is a diffraction grating, visibility of the transdermal administration device is appropriately improved since the raised and/or recessed structure 13 exhibits a structural color.
(5) In the configuration in which the transdermal administration device includes the adhesive sheet 30, visibility of a portion which serves as an administration section in the transdermal administration device having the adhesive sheet 30 is improved.
(6) Typically, the plurality of projections 12 is located at an easily recognizable position on the first surface 11S such as the center of the first surface 11S. In the configuration in which the region of the second surface 11T where the raised and/or recessed structure 13 is located overlaps with at least part of the region of the first surface 11S where a group composed of the plurality of projections 12 is located as viewed in the direction perpendicular to the first surface 11S, the raised and/or recessed structure 13 is readily recognized since the raised and/or recessed structure 13 is located in a portion which overlaps with an easily recognizable position on the first surface 11S. Accordingly, visibility of the transdermal administration device is appropriately improved. Further, in the configuration in which the region of the second surface 11T where raised and/or recessed structure 13 is located overlaps with the entire region of the first surface 11S where a group composed of the plurality of projections 12 is located as viewed in the direction perpendicular to the first surface 11S, visibility of the transdermal administration device is further improved since the raised and/or recessed structure 13 is located at a more easily recognizable position.

### [Modified Examples]

The above embodiment and modified examples can be implemented with modifications as described below.
- In the configuration in which a pattern formed by the plurality of raised and/or recessed elements 14 is finer than a pattern formed by the plurality of projections 12, and the optical properties of the portion of the microneedle 10 where the raised and/or recessed structure 13 is disposed are different from those of the portion where the raised and/or recessed structure 13 is not disposed, at least one of the following requirements does not need to be satisfied: the distance between the raised and/or recessed elements is smaller than the distance between the projections, the width Dr of the raised and/or recessed elements 14 is smaller than the width Dt of the projections 12, and the area of the raised and/or recessed elements 14 as viewed in the direction perpendicular to the second surface 11T is smaller than the area of the projections 12 as viewed in the direction perpendicular to the first surface 11S. For example, when viewed in the direction perpendicular to the second surface 11T of the substrate 11, the raised and/or recessed elements 14 may have a linear shape, the raised and/or recessed elements 14 may extend linearly in one direction, connecting both ends of the raised and/or recessed structure 13, or the plurality of raised and/or recessed elements 14 may be arranged at a desired distance therebetween. In this case, a stripe pattern is formed by the plurality of raised and/or recessed elements 14. Alternatively, a grid pattern may be formed by the plurality of raised and/or recessed elements 14 by alternately arranging the linearly extending raised and/or recessed elements 14. That is, the raised and/or recessed elements 14 are constituent elements of the pattern, and the plurality of raised and/or recessed elements 14 may be independent from each other or may be connected to each other.

Further, as shown in Fig. 13, gaps may not be provided between the plurality of raised and/or recessed elements 14. In this case, certain raised and/or recessed elements 14 are referred to as the first raised and/or recessed elements and the raised and/or recessed elements 14 adjacent to the first raised and/or recessed elements are referred to as the second raised and/or recessed elements. The distance between the raised and/or recessed elements, which is the distance between the first raised and/or recessed elements and the second raised and/or recessed elements is the width of each of the first raised and/or recessed elements in the direction in which the first raised and/or recessed elements and the second raised and/or recessed elements are arranged, that is, the maximum length of the first raised and/or recessed elements in this direction. Further, Fig. 13 shows an example in which the raised and/or recessed elements 14 having shapes different from each other are irregularly arranged.

- The shape of each of the projections 12 of the administration section is not limited to a needle-shape, that is, a shape extending in the direction perpendicular to the first surface 11S of the substrate 11. The shape of the each of the projections 12 may be a blade shape, that is, a linear shape in which the projection 12 extends in one extending direction which is a direction extending along the first surface 11S of the substrate 11, and a distal end of the projection 12 extends in a direction which is not perpendicular to the first surface 11S of the substrate 11, for example, in a direction linearly extending along the extending direction. For example, the projection 12 may be formed as a triangular prism shape extending along the extending direction while one of three rectangular side surfaces of the triangular prism is in contact with the substrate 11 and the side of the triangular prism that partitions the other two side surfaces serves as a distal end of the projection 12.

### [Examples]

The aforementioned transdermal administration device will be described by using specific examples and comparative examples.

### <Example 1>

### Step 1. Production of intaglio plate

First, an original plate for a microneedle was fabricated by micromachining. A silicon substrate was used for a material forming the original plate in which four projections were arrayed in a matrix of 2 x 2 at a pitch of 4 mm. Each of the projections was formed in a regular quadrangular pyramid (height: 120 µm, bottom: 38 µm x 38 µm). The four projections were disposed in a square region, each side of which has a length of approximately 10 mm.

Then, the shape of the projections of the original plate was transferred by electroforming to thereby fabricate a nickel intaglio plate having a reversed pattern of raised and recessed portions of the original plate.

### Step 2. Production of substrate and projections

Then, the intaglio plate was filled with polycarbonate by injection molding. The molded product was then demolded to thereby obtain a structure having the transparent substrate and projections made of polycarbonate. The substrate had a thickness of 500 µm, and each of the projections had a length of 120 µm.

### Step 3. Production of raised and/or recessed structure

Then, the recessed elements were formed as the raised and/or recessed elements by laser processing on the surface of the substrate opposite to the surface on which the projections are disposed to thereby form the raised and/or recessed structure.

Thus, the transdermal administration device, which is the microneedle of Example 1, in which a group of the plurality of projections is disposed in a square region of 10 mm x 10 mm at the center of the first surface of the substrate and the raised and/or recessed structure are disposed on the second surface of the substrate was obtained.

### <Example 2>

### Step 1. Production of intaglio plate

First, an original plate for a microneedle was fabricated by micromachining. A silicon substrate was used for a material forming the original plate in which four projections were arrayed in a matrix of 2 x 2 at a pitch of 4 mm. Each of the projections was formed in a regular quadrangular pyramid (height: 120 µm, bottom: 38 µm x 38 µm). The four projections were disposed in a square region, each side of which has a length of approximately 10 mm.

Then, the shape of the projections of the original plate was transferred by electro forming to thereby fabricate a nickel intaglio plate having a reversed pattern of raised and recessed portions of the original plate.

### Step 2. Production of substrate and projections and raised and/or recessed structure

Then, the microneedle was produced by heat and pressure molding. Specifically, polycarbonate was disposed on the intaglio plate, and the intaglio plate was heated to melt the polycarbonate. The polycarbonate was then pressed against the intaglio plate by using a sandblasted metal plate. Subsequently, the intaglio plate was cooled, and the molded product was removed from the intaglio plate to thereby obtain the transdermal administration device, which is the microneedle of Example 2. In the above production method, a sandblasted metal plate was used as a member for pressing the polycarbonate against the intaglio plate to thereby form the raised and/or recessed structure on the second surface of the substrate in the same step as the step of forming the substrate and the projections.

The obtained transparent microneedle made of polycarbonate had the substrate with a thickness of 500 µm and projections with a length of 120 µm.

Thus, the transdermal administration device of Example 2, in which a group of the plurality of projections is disposed in a square region of 10 mm x 10 mm at the center of the first surface of the substrate and the raised and/or recessed structure are disposed on the second surface of the substrate was obtained.

### <Comparative Examples>

### Step 1. Production of intaglio plate

First, an original plate for a microneedle was fabricated by micromachining. A silicon substrate was used for a material forming the original plate in which four projections were arrayed in a matrix of 2 x 2 at a pitch of 4 mm. Each of the projections was formed in a regular quadrangular pyramid (height: 120 µm, bottom: 38 µm x 38 µm). The four projections were disposed in a square region, each side of which has a length of approximately 10 mm.

Then, the shape of the projections of the original plate was transferred by electroforming to thereby fabricate a nickel intaglio plate having a reversed pattern of raised and recessed portions of the original plate.

### Step 2. Production of substrate and projections

Then, the intaglio plate was filled with polycarbonate by injection molding. The molded product was then demolded to thereby obtain a structure having the transparent substrate and projections made of polycarbonate. The substrate had a thickness of 500 µm, and each of the projections had a length of 120 µm.

Thus, the transdermal administration device, which is the microneedle of the comparative example, in which a group of the plurality of projections is disposed in a square region of 10 mm x 10 mm at the center of the first surface of the substrate without having the raised and/or recessed structure was obtained.

### <Evaluation>

The transdermal administration devices of the examples and the comparative example were placed on the black substrate with the tip of the projections oriented upward and visually observed in the direction facing the first surface.

For the transdermal administration devices of the examples, a translucent portion was observed in the lower part of the substrate in both Example 1 and Example 2. As a result, the position of the transdermal administration device on the black substrate was easily observed.

On the other hand, for the transdermal administration device of the comparative example, visual observation of the transdermal administration device, and thus identification of the position of the transdermal administration device on the black substrate was difficult. The position which seems to have the projections was managed to be recognized when the transdermal administration device was observed from a position close to the black substrate.

### [Reference Signs List]

10...Microneedle, 11...Substrate, 11S...First surface, 11T...Second surface, 12...Projection, 13...Raised and/or recessed structure, 14...Raised and/or recessed elements, 15...Raised and/or recessed area, 20...Transdermal administration device, 30...Adhesive sheet

## Claims

1. A transdermal administration device (20) comprising:
a substrate (11) having a first surface (11S) and a second surface (11T) which is a surface opposite to the first surface (11S);
a plurality of projections (12) protruding from the first surface (11S), the plurality of projections (12) being provided for piercing an administration target; and
a raised and/or recessed structure (13) composed of a plurality of raised and/or recessed elements (14), the plurality of raised and/or recessed elements (14) including at least either raised elements raised from the second surface (11T) or recessed elements recessed from the second surface (11T), wherein
the substrate (11), the projections (12) and the raised and/or recessed structure (13) are made of transparent material, and
the plurality of raised and/or recessed elements (14) forms a pattern as viewed in a direction perpendicular to the second surface (11T) which is finer than a pattern formed by the plurality of projections (12) as viewed in a direction perpendicular to the first surface (11S).

2. The transdermal administration device (20) according to claim 1, wherein
a distance between the respective ends of two of the projections (12) adjacent to each other as viewed in the direction perpendicular to the first surface (11S), which is the distance between the respective ends oriented in the same direction in a direction of an array of the projections (12), is an inter-projection distance,
a distance between the respective ends of two of the raised and/or recessed elements (14) adjacent to each other as viewed in the direction perpendicular to the second surface (11T), which is the distance between the respective ends oriented in the same direction in a direction of an array of the raised and/or recessed elements (14), is an inter-raised and/or recessed element distance, and
the inter-raised and/or recessed elements (14) distance is smaller than the inter-projection distance.

3. The transdermal administration device (20) according to claim 1 or 2, wherein a maximum length of each of the raised and/or recessed elements (14) in a direction parallel with the second surface (11T) is smaller than a maximum length of each of the projections (12) in a direction parallel with the first surface (11S).

4. The transdermal administration device (20) according to any one of claims 1 to 3, wherein an area occupied by one of the raised and/or recessed elements (14) as viewed in the direction perpendicular to the second surface (11T) is smaller than an area occupied by one of the projections (12) as viewed in the direction perpendicular to the first surface (11S).

5. The transdermal administration device (20) according to any one of claims 1 to 4, wherein a maximum length of each of the raised and/or recessed elements (14) in a thickness direction of the substrate (11) is smaller than a maximum length of each of the projections (12) in a thickness direction of the substrate (11).

6. The transdermal administration device (20) according to any one of claims 1 to 5, wherein the plurality of raised and/or recessed elements (14) is a diffraction grating.

7. The transdermal administration device (20) according to any one of claims 1 to 6, further comprising an adhesive sheet (30) having an adhesive layer (32) bonded to the second surface (11T) of the substrate (11), wherein the adhesive layer (32) extends outward from the substrate (11) as viewed in a direction perpendicular to the first surface (11S) of the substrate (11).

## Patentansprüche

1. Transdermale Verabreichungsvorrichtung (20), umfassend:
ein Substrat (11) mit einer ersten Oberfläche (11S) und einer zweiten Oberfläche (11T), die eine Oberfläche entgegengesetzt zu der ersten Oberfläche (11S) ist;
eine Vielzahl von Vorsprüngen (12), die von der ersten Oberfläche (11S) vorstehen, wobei die Vielzahl von Vorsprüngen (12) zum Durchdringen eines Verabreichungsziels vorgesehen ist; und
eine erhabene und/oder vertiefte Struktur (13), die aus einer Vielzahl von erhabenen und/oder vertieften Elementen (14) besteht, wobei die Vielzahl von erhabenen und/oder vertieften Elementen (14) mindestens entweder von der zweiten Oberfläche (11T) erhabene Elemente oder von der zweiten Oberfläche (11T) vertiefte Elemente beinhaltet, wobei
das Substrat (11), die Vorsprünge (12) und die erhabene und/oder vertiefte Struktur (13) aus transparentem Material hergestellt sind, und
die Vielzahl von erhabenen und/oder vertieften Elementen (14) ein Muster bildet, wenn man es in einer Richtung senkrecht zur zweiten Oberfläche (11T) betrachtet, das feiner ist als ein Muster, das durch die Vielzahl von Vorsprüngen (12) in einer Richtung senkrecht zur ersten Oberfläche (11S) betrachtet wird.

2. Transdermale Verabreichungsvorrichtung (20) nach Anspruch 1, wobei
ein Abstand zwischen den jeweiligen Enden von zwei der Vorsprünge (12), die in der Richtung senkrecht zur ersten Oberfläche (11S) gesehen nebeneinander liegen, was der Abstand zwischen den jeweiligen Enden ist, die in der gleichen Richtung in Richtung einer Anordnung der Vorsprünge (12) ausgerichtet sind, ein zwischen den Vorsprüngen liegender Abstand ist,
ein Abstand zwischen den jeweiligen Enden von zwei der erhabenen und/oder vertieften Elemente (14), die in der Richtung senkrecht zur zweiten Oberfläche (11T) gesehen nebeneinander liegen, der der Abstand zwischen den jeweiligen Enden ist, die in der gleichen Richtung in einer Richtung einer Anordnung der erhabenen und/oder vertieften Elemente (14) ausgerichtet sind, ein Abstand zwischen den erhabenen und/oder vertieften Elementen ist, und
der Abstand zwischen erhabenen und/oder vertieften Elementen (14) kleiner ist als der Abstand zwischen den Vorsprüngen.

3. Transdermale Verabreichungsvorrichtung (20) nach Anspruch 1 oder 2, wobei eine maximale Länge jedes der erhabenen und/oder vertieften Elemente (14) in einer Richtung parallel zu der zweiten Oberfläche (11T) kleiner als eine maximale Länge jedes der Vorsprünge (12) in einer Richtung parallel zu der ersten Oberfläche (11S) ist.

4. Transdermale Verabreichungsvorrichtung (20) nach einem der Ansprüche 1 bis 3, wobei ein Bereich, der von einem der erhabenen und/oder vertieften Elemente (14) in der Richtung senkrecht zur zweiten Oberfläche (11T) eingenommen wird, kleiner ist als ein Bereich, der von einem der Vorsprünge (12) in der Richtung senkrecht zur ersten Oberfläche (11S) eingenommen wird.

5. Transdermale Verabreichungsvorrichtung (20) nach einem der Ansprüche 1 bis 4, wobei eine maximale Länge jedes der erhabenen und/oder vertieften Elemente (14) in einer Dickenrichtung des Substrats (11) kleiner ist als eine maximale Länge jedes der Vorsprünge (12) in einer Dickenrichtung des Substrats (11).

6. Transdermale Verabreichungsvorrichtung (20) nach einem der Ansprüche 1 bis 5, wobei die Vielzahl der erhabenen und/oder vertieften Elemente (14) ein Diffraktionsgitter ist.

7. Transdermale Verabreichungsvorrichtung (20) nach einem der Ansprüche 1 bis 6, ferner umfassend eine Klebefolie (30) mit einer mit der zweiten Oberfläche (11T) des Substrats (11) verbundenen Klebeschicht (32), wobei sich die Klebeschicht (32) vom Substrat (11) aus gesehen in einer Richtung senkrecht zur ersten Oberfläche (11S) des Substrats (11) nach außen erstreckt.

## Revendications

1. Dispositif d'administration transdermique (20) comprenant :
un substrat (11) ayant une première surface (11S) et une deuxième surface (11T) qui est une surface opposée à la première surface (11S) ;
une pluralité de protubérances (12) faisant saillie à partir de la première surface (11S), la pluralité de protubérances (12) étant prévue pour percer une cible d'administration ; et
une structure en élévation et/ou en renfoncement (13) composée d'une pluralité d'éléments en élévation et/ou en renfoncement (14), la pluralité d'éléments en élévation et/ou en renfoncement (14) comprenant au moins soit des éléments en élévation s'élevant à partir de la deuxième surface (11T) soit des éléments en renfoncement renfoncés par rapport à la deuxième surface (11T),
dans lequel
le substrat (11), les protubérances (12) et la structure en élévation et/ou en renfoncement (13) sont faits d'un matériau transparent, et
la pluralité d'éléments en élévation et/ou en renfoncement (14) forme un motif, tel que vu dans une direction perpendiculaire à la deuxième surface (11T), qui est plus fin qu'un motif formé par la pluralité de protubérances (12) tel que vu dans une direction perpendiculaire à la première surface (11S).

2. Dispositif d'administration transdermique (20) selon la revendication 1, dans lequel
la distance entre les extrémités respectives de deux des protubérances (12) adjacentes l'une à l'autre, telle que vue dans la direction perpendiculaire à la première surface (11S), qui est la distance entre les extrémités respectives orientées dans la même direction et dans la direction d'un réseau des protubérances (12), est la distance inter-protubérances,
la distance entre les extrémités respectives de deux des éléments en élévation et/ou en renfoncement (14) adjacents l'un à l'autre, telle que vue dans la direction perpendiculaire à la deuxième surface (11T), qui est la distance entre les extrémités respectives orientées dans la même direction et dans la direction d'un réseau des éléments en élévation et/ou en renfoncement (14), est la distance inter-éléments en élévation et/ou en renfoncement, et
la distance inter-éléments en élévation et/ou en renfoncement (14) est inférieure à la distance inter-protubérances.

3. Dispositif d'administration transdermique (20) selon la revendication 1 ou 2, dans lequel la longueur maximale de chacun des éléments en élévation et/ou en renfoncement (14) dans une direction parallèle à la deuxième surface (11T) est inférieure à la longueur maximale de chacune des protubérances (12) dans une direction parallèle à la première surface (11S).

4. Dispositif d'administration transdermique (20) selon l'une quelconque des revendications 1 à 3, dans lequel la surface occupée par l'un des éléments en élévation et/ou en renfoncement (14), telle que vue dans la direction perpendiculaire à la deuxième surface (11T), est inférieure à la surface occupée par l'une des protubérances (12), telle que vue dans la direction perpendiculaire à la première surface (11S) .

5. Dispositif d'administration transdermique (20) selon l'une quelconque des revendications 1 à 4, dans lequel la longueur maximale de chacun des éléments en élévation et/ou en renfoncement (14) dans la direction de l'épaisseur du substrat (11) est inférieure à la longueur maximale de chacune des protubérances (12) dans la direction de l'épaisseur du substrat (11).

6. Dispositif d'administration transdermique (20) selon l'une quelconque des revendications 1 à 5, dans lequel la pluralité d'éléments en élévation et/ou en renfoncement (14) est une grille de diffraction.

7. Dispositif d'administration transdermique (20) selon l'une quelconque des revendications 1 à 6, comprenant en outre une feuille adhésive (30) ayant une couche adhésive (32) collée à la deuxième surface (11T) du substrat (11), dans lequel la couche adhésive (32) s'étend en-dehors depuis le substrat (11), telle que vue dans une direction perpendiculaire à la première surface (11S) du substrat (11).
